# EUROPÄISCHE PATENTSCHRIFT

(11) **EP 1 093 361 B1**
(45) Veröffentlichungstag und Bekanntmachung des Hinweises auf die Patenterteilung: **02.05.2003**
(21) Anmeldenummer: 99934611.7
(22) Anmeldetag: 07.07.1999
(51) Int. Cl.: A61K 9/70

(54) **STEROIDHALTIGES PFLASTER, VERFAHREN ZU SEINER HERSTELLUNG UND VERWENDUNG**
PLASTER WHICH CONTAINS STEROIDS, AND A METHOD FOR THE PRODUCTION AND USE THEREOF
EMPLATRE CONTENANT DES STEROIDES, PROCEDE PERMETTANT DE LE PRODUIRE ET SON UTILISATION

(30) Priorität: 09.07.1998 DE 19830651
(43) Veröffentlichungstag der Anmeldung: 25.04.2001
(73) Patentinhaber: LTS LOHMANN THERAPIE-SYSTEME GmbH & CO.KG, 56626 Andernach (DE)
(72) Erfinder: BERTHOLD, Achim, D-56626 Andernach (DE); MÜLLER, Walter, D-56564 Neuwied (DE)
(74) Vertreter: Flaccus, Rolf-Dieter, Dr.
(86) Internationale Anmeldenummer: EP9904756
(87) Internationale Veröffentlichungsnummer: WO00002540

(56) Entgegenhaltungen:
- EP-A- 0 464 573
- EP-A- 0 848 960
- WO-A-95/31193

## Beschreibung

Die vorliegende Erfindung betrifft eine medizinische Zusammensetzung zur transdermalen Applikation eines Estrogens in Kombination mit einem Gestagen.

Bei der kutanen Applikation von Wirkstoffen wird die systemische Wirkung der Arzneistoffe angestrebt. Es eignen sich hierfür wegen der begrenzten Permeabilität der Haut bevorzugt Substanzen, die in einer niedrigen Dosierung appliziert werden (Tagesdosen bis 10 mg).

Die kutane Anwendung ist dann sinnvoll, wenn nach oraler Gabe ein großer Wirkstoffanteil bei der ersten Passage durch die Schleimhäute des Magen-Darm-Kanals metabolisiert bzw. durch die Leber zurückgehalten wird (First-Pass-Effekt) und/oder wenn der Wirkstoff eine niedrige Plasmahalbwertszeit besitzt. Demgegenüber sind Stoffe mit einem hohen Allergisierungspotential sowie solche, die lokal irritierend wirken, ungeeignet. Werden die Grundvoraussetzungen erfüllt, dann stellt die kutane Applikation eine Alternative zur oralen Gabe dar.
Die Erfindung betrifft eine wirkstoffhaltige Vorrichtung, die einen oder mehrere Arzneistoffe in einer vorausbestimmten Rate kontinuierlich über einen festgelegten Zeitraum an einen festgelegten Ort freigibt. Ein derartige Vorrichtung ist durch ein exaktes Behandlungsprogramm charakterisiert und wird als therapeutisches System bezeichnet. Da das erfindungsgemäße System als Pflaster auf die Haut geklebt wird, um einen systemischen Effekt zu erzielen, spricht man in diesen Zusammenhang von einem transdermalen therapeutischen System (TTS).

Die Erfindung betrifft eine Zubereitung mit einem hohen Wirkungsgrad, das heißt, die Zubereitung führt zu einer hohen Bioverfügbarkeit der Arzneistoffe. Dies wird dadurch erreicht, daß die präsystemische Elimination durch Umgehen des Verdauungstraktes stark reduziert wird, wobei die Wirksamkeit der Arzneistoffe unabhängig von der Magenentleerungsrate und der intestinalen Motilität ist.

Die Wirkung der Arzneistoffe kann durch einfache Entfernung der Zubereitung jederzeit - mit einer gewissen Zeitverzögerung - abgebrochen werden. Die Plasmakonzentration kann ohne Spitzen und Täler im therapeutischen Bereich eingestellt werden. Infolgedessen zeichnet sich die Zubereitung durch eine Steuerbarkeit des Absorptionsgeschehens aus.

Die erfindungsgemäße Zubereitung hat eine hohe Zuverlässigkeit in Hinblick auf die Befolgung des Therapieplans durch den Patienten, da die Applikationsfrequenz im Vergleich zu konventionellen oralen Arzneiformen stark reduziert ist. Ferner kann die applizierte Wirkstoffmenge in der Regel vermindert werden. Somit sind dosisabhängige Nebenwirkungen ebenfalls reduziert oder werden gar vermieden. Daraus resultiert eine erhöhte Therapiesicherheit.

Estradiol, Estron (Estrogene) und Progesteron (Gestagen) sind die natürlichen weiblichen Sexualhormone. Die Sexualhormone dienen der Ausbildung der primären und sekundären Geschlechtsmerkmale. Sie haben einen Einfluß auf das Wachstum und den Körperaufbau sowie auf den Wasser- und Mineralhaushalt. Ferner prägen die Sexualhormone den Ablauf des menstruellen Zyklus bei der Frau.

Die natürlichen Sexualhormone, deren Derivate sowie Strukturanaloga werden zur hormonalen Kontrazeption, zur Substitutitherapie oder zur Behandlung verschiedener Erkrankungen eingesetzt.

Ein Hauptanwendungsgebiet der Sexualhormone liegt auf dem Gebiet der postmenopausalen Hormonsubstitution. Die Substitution dient der Vorbeugung klimakterischer Beschwerden (Hitzewallungen, Schwindel, Tachykardien, Schwitzen, Angstgefühl, Reizbarkeit, Konzentrationsschwächen, Schlafstörungen etc.). Ferner sollen Veränderungen an den Harn- und Geschlechtsorganen, kardiovaskuläre Veränderungen bedingt durch Hyperlipoproteinämien, Hautatrophien oder Osteoporose sowie weitere krankhafte Erscheinungen verhindert werden. Zu diesem Zweck wird ein Estrogen in Kombination mit einem Gestagen verabreicht.

Beispielsweise können folgende Estrogene eingesetzt werden: 17-beta-Estradiol, 17-alpha-Estradiol, 17-beta-Estradiolcypionat, 17-beta-Ethinylestradiol, 3,17-beta-Estradioldienanthat, 17-beta-Estradiolvalerate, 17-beta-Estradiolbenzoat, 17-beta-Estradiolundecylat, 17-Deacetylnorgestimat, Norgestimat, Mestranol und Quinestrol. Die genannten Estrogene sind durch eine aromatische Hydroxylgruppe bzw. deren Ether charakterisiert.

Da das natürliche Gestagen Progesteron für die Substitutionstherapie unzureichende pharmakokinetische Eigenschaften aufweist, wurden zahlreiche Abwandlungsprodukte synthetisiert. Hier sind beispielhaft zu nennen: 19-Norprogesteron, Norethisteronacetat, Norethisteron, Ethisteron, Melengestrol, Norgestrel, Levonorgestrel, Gestoden, Hydroxyprogesteroncapronat, Medroxyprogesteronacetat, Ethynodioldiacetat, 17-alpha-Hydroxyprogesteron, Megestrolacetat, Lynestrenol, Desogestrel, Allylestrenol, Chlormadinon und Chlormadinonacetat. Typisches Strukturmerkmal der meisten Verbindungen ist eine 3-Keto-4-en Struktur.

Wird Estradiol oral gegeben, wird es aufgrund seiner schlechten Wasserlöslichkeit nur zu einen geringen Teil absorbiert. Der absorbierte Anteil unterliegt einem hohen First-Pass-Effekt. Dabei werden zahlreiche Metaboliten gebildet, die keine Estrogenwirkung mehr besitzen und zu Nebenwirkungen führen. Ferner führt die orale Gabe zu einer unphysiologischen Fluktuation des Hormonblutspiegels. Bedingt durch den First-Pass-Effekt müssen zudem hohe Estradiolmengen gegeben werden. Dadurch werden weitere Nebenwirkungen bedingt.

Die ideale Applikation für Estradiol wäre eine langsame intravenöse Infusion. Dies ist jedoch nicht praktikabel. Durch die transdermale Gabe lassen sich fast ideale Bedingungen erreichen. Dabei wird der First-Pass-Effekt umgangen und Plasmakonzentrationen erzielt, die dem physiologischen Hormonblutspiegel einer prämenopausalen Frau entsprechen (40-60 pg/ml). Ein weiterer Vorteil der transdermalen Gabe gegenüber der oralen Applikation ist, daß die tägliche Dosis auf 50 µg/Tag gesenkt werden kann.

Das Hauptrisiko, das mit der postmenopausalen Estradiolgabe verbunden ist, ist die Hyperstimulation des Endometriums, verbunden mit einem erhöhten Risiko einer Hyperplasie bzw. einer Entartung. Darüber hinaus treten unter Monotherapie mit Estradiol vermehrt Störungen der Regelblutung auf. Um diese Risiken zu mindern, ist es sinnvoll, Estradiol in Kombination mit einem Gestagen zu geben. Bei der transdermalen Gabe sind täglich zur Vermeidung eine Hyperplasie ca. 200-300 µg Norethisteronacetat oder ein entsprechendes Äquivalent notwendig. Im Vergleich hierzu sind bei der peroralen Gabe 0,7-1 mg/Tag erforderlich [Wiseman, L. R. and McTavish, D., Transdermal Estradiol/Norethisterone: A Review of its Pharmacological Properties and Clinical Use in Postmenopausal Women, Drug & Aging, Vol. 4, No. 3, 1994, 238-256].

Kombinierte TTS mit einer Zusammensetzung zur transdermalen Applikation eines Estrogens, insbesondere von Estradiol, in Kombination mit einem Gestagen, insbesondere von Norethisteronacetat sind bereits bekannt und auf dem Markt erhältlich (z.B. Estracomb®). Von Nachteil ist deren komplexer Aufbau. So enthält Estracomb® zwei Kompartimente, die als Wirkstoffreservoir dienen. Ferner ist eine Steuermembran zur kontrollierten Wirkstoffabgabe enthalten. Durch die raumfordernden Kompartimente ist das System sehr dick und daher unkomfortabel zu tragen.

Beispiele für einfach aufgebaute Matrixsysteme zur transdermalen Applikation von Estradiol in Kombination mit einem Gestagen sind in der EP 0 695 177 B1 beschrieben. In der WO 96/40087 ist ein Matrixsystem auf Grundlage eines vernetzten Acrylatpolymers zur transdermalen Applikation von Estradiol beschrieben.

EP-A-0 848 960 offenbart ein Haft- und Bindemittel für dermale oder transdermale Therapiesysteme bestehend aus (a1) 55-99.9 Gew.-% eines (Meth)acrylatcopolymers, (a2) 0.1-45 Gew.-% eines säuregruppenhaltigen Arylat- oder (Meth)acrylat Polymeren oder Copolymeren und (b) 25-80 Gew.-%, bezogen auf die Summe von (a1) und (a2), eines Weichmachers. Als eingesetzte Arzneistoffe werden Hormone erwähnt.

Bei der Lagerung steroidhaltiger TTS vom Matrixtyp treten unter anderem zwei Stabilitätsprobleme auf: die Wirkstoffe können rekristallisieren oder abgebaut werden.

Rekristallisation tritt dann in Erscheinung, wenn die Sättigungskonzentration des entsprechenden Wirkstoffes überschritten wird. Die Überschreitung der Sättigungskonzentration kann die Folge einer Modifikationsänderung sein. So neigt Estradiol durch Aufnahme von Kristallwasser zur Bildung eines Semihydrates, welches aufgrund seiner geringeren Löslichkeit rekristallisiert. Ferner besteht die Möglichkeit der Bildung von Mischkristallen. Hierbei ist die Löslichkeit der Mischkristalle geringer als die Sättigungslöslichkeit der einzelnen Komponenten. Infolge der Rekristallisation nimmt die thermodynamische Aktivität und die Permeationsrate der Wirkstoffe durch die Haut ab. Die therapeutische Wirksamkeit der Zubereitung wird dadurch gefährdet.

Um die Rekristallisation des Estradiols zu verhindern, sind verschiedene Möglichkeiten beschrieben. Das in dem US-Patent 5,676,968 beschriebene System enthält in dem wirkstoffhaltigen Kompartiment Hilfsstoffe, die als "Kristallisationshemmer" bezeichnet werden und dem Kristallisationsprozeß entgegenwirken sollen. Insbesondere werden hier Siliciumdioxid und makromolekulare Substanzen, wie Polyvinylpyrrolidon oder dessen Copolymere mit Vinylacetat, genannt. In dem US-Patent 5,711,962 und in der WO 97/23227 ist der Zusatz von Octyldodecanol zur Verhinderung der Kristallisation beschrieben. In der WO 96/05814 ist ein System beschrieben, das wasserfreies Glycerin als Bestandteil der Matrix enthält. Wasserfreies Glycerin ist in jedem Verhältnis mit Wasser mischbar, stark hygroskopisch und kann als wasserentziehendes Mittel eingesetzt werden. Wird wasserfreies Glycerin zusammen mit Estradiol-Semihydrat gelagert, so kann es diesem das Kristallwasser entziehen. Wasserfreies Glycerin ist damit ein geeignetes Mittel, um die Rekristallisation während der Lager zu verhindern. In der WO 96/05815 wird der Zusatz von wasserbindenden mineralischen Bestandteilen zur wirkstoffhaltigen Matrix beschrieben, so daß die Rekristallisation von Estradiol-Semihydrat verhindert wird. Als mineralische Bestandteile werden zum Beispiel das Anhydrat des Calciumsulfates, Zinkoxid, Magnesiumoxid, Siliciumdioxid, Silicagel, Talkum und weiter Substanzen genannt. In der DE 42 37 453 ist die Verwendung von Trockenmitteln in der Primärverpackung vorgeschlagen.
EP 0 186 019 A1 beschreibt die Verwendung eines wasserquellbaren Polymers zur Kristallisationsverzögerung des oberhalb seiner Sättigungskonzentration vorliegenden Wirkstoffes. In der EP 0 695 177 B1 ist ein System beschrieben, das Estradiol in einer Konzentration nahe der Sättigung enthält. Erst nach dem Aufkleben auf die Haut wird die Sättigungskonzentration durch Wasseraufnahme überschritten und dadurch die thermodynamische Aktivität erhöht. Hierbei bestimmt alpha-Tocopherol den Grad der Übersättigung der hydratisierten Matrix und damit die Diffusion des Wirkstoffes durch die Haut. Alpha-Tocopherol dient in diesem Zusammenhang zur Verbesserung der Wirkstofflöslichkeit und zur Verhinderung der Rekristallisation während der Lagerung.

Steroide (Hormone und Corticoide) werden in Abhängigkeit von den eingesetzten Hilfsstoffen mehr oder weniger stark während der Lagerung abgebaut. Hierbei müssen verschiedene Abbaumechanismen in Betracht gezogen werden. Zum einen treten Verseifungsreaktionen in Erscheinung, die zu hydrophileren Verbindungen führen und zum andern Oxidationsreaktionen, die zu unwirksamen Produkten führen. Insbesondere Steroide, die eine 3-Keto-4-en Partialstruktur aufweisen, sind sehr empfindlich. Für diese Instabilität werden unter anderen sauer reagierende Gruppen verantwortlich gemacht. So beschreibt WO 97/03629 ein System mit einem Träger, der keine Säurefunktionen aufweist und solche auch nicht während der Lagerung bildet. In der DE 195 48 332 A1 ist ein Hormonpflaster mit Norethisteronacetat beschrieben, das eine gute Lagerstabilität besitzt. Hierbei wird die Stabilität durch Einsatz eines bestimmten Polymergemisches erreicht. In WO 97/23227 wird beschrieben, daß der Abbau des Norethisteronacetats von dem Feuchtigkeitsgehalt der Matrix abhängig ist. Demzufolge ist es von Vorteil, wenn bei der Herstellung mit getrockneter Luft gearbeitet wird und wenn ein Trockenmittel (z. B. Natrium- oder Calciumsulfat) in die Primärverpackung integriert wird. Ferner ist beschrieben, daß die Stabilität des Norethisteronacetats verbessert wird, wenn während der Herstellungen der Wirkstoff in einem Lösungsmittelgemisch, bestehend aus Methylethylketon und Ethanol, gelöst wird.

Neben den genannten Stabilitätsproblemen muß die therapeutische Wirksamkeit der TTS sichergestellt werden. Die therapeutische Wirksamkeit wird durch das Ausmaß der Hautpermeation bestimmt. Zur Sicherstellung einer ausreichenden Permeation werden häufig Enhancer eingesetzt. Zum Beispiel ist in dem US-Patent 5,676,968 der Einsatz von 1,2-Propandiol und Isopropylmyristat zur Steigerung der Estradiol-Permeation beispielhaft beschrieben. Die EP 0 811 381 A1 beschreibt den Einsatz von Fettalkoholen in Kombination mit einem Diethylenglycolmonoalkylether als Enhancer für Estradiol und Norethisteronacetat. Das US-Patent 5,686,097 nennt die Verwendung von Monoglyceriden und Lactatestern zur Förderung der Penetration einer Estrogen/Gestagen-Kombination.

Der Einsatz von Enhancern ist jedoch nicht immer unproblematisch. Viele der eingesetzten Substanzen führen zu Hautirritationen. Zur Minderung dieser Irritationen werden oft weitere Hilfsstoffe eingesetzt, die dem entgegenwirken sollen. Hier sind beispielsweise Glycerol und Polyglycerolether zu nennen. Neben dem lindernden Effekts wirkt sich Glycerol ebenfalls positiv auf die Permeationsrate aus. Nachteilig ist, daß Glycerin die Kohäsion einer Polyacrylatmatrix stark senkt. Eine Verschlechterung der Kohäsion äußert sich in einem erhöhten "tack", einem erhöhten "kalten Fluß", einem Transfer von Kleber auf die Schutzfolie oder in einem Verbleib von Kleberresten auf der Haut nach Entfernung des Systems. Zur generellen Verbesserung der Kohäsion von Systemen mit einem hohen Gehalt an flüssigen bzw. weichmachenden Komponenten ist in dem US-Patent 5,306,503 der Zusatz von sogenannten Filmbildnern genannt.

Aufgabe der vorliegenden Erfindung war die Bereitstellung eines einfach aufgebauten TTS, eines sogenannten Matrixsystems, mit einer kostengünstigen Zusammensetzung zur transdermalen Applikation eines Estrogens, insbesondere von Estradiol, in Kombination mit einem Gestagen, insbesondere von Norethisteronacetat, welches eine effektive Arzneimitteltherapie der klimakterischen Beschwerden gewährleistet.
Matrixsysteme sind dadurch gekennzeichnet, daß die Wirkstoffe fein verteilt (gelöst oder dispergiert) in einer Polymermatrix vorliegen. Hierbei hat die Matrix Reservoir- und Klebefunktion. Während bei einem Membransystem die transdermale Wirkstoffaufnahme weitgehend durch die integrierte Steuennembran reguliert wird, wird diese Funktion bei Matrixsystemen von der Haut übernommen. Die Herstellung der wirkstoffhaltigen Matrix gestaltet sich sehr einfach.

Diese Aufgabe wird gelöst durch eine selbstklebende Zusammensetzung auf Polyacrylatbasis zur transdermalen Applikation gemäß den Merkmalen des Hauptanspruches. Bevorzugte und vorteilhafte Ausführungsformen weisen die Merkmale der Unteransprüche auf.

Zusammensetzungen auf Polyacrylatbasis verfügen über ein sehr geringes allergenes Potential und sind daher auch für eine längeren Applikation geeignet. Die Klebrigkeit der Polymere kommt dabei allein aufgrund der Molekulargewichtsverteilung und der verwendeten Monomere zustande.

Zur Herstellung erfindungsgemäßer Zusammensetzungen auf Polyacrylatbasis werden Acrylsäure und/oder Alkylacrylsäure, insbesondere Methacrylsäure oder ihre Derivate, insbesondere die Alkylester, eingesetzt. Unter den Alkylestern der Acrylsäure und/oder Methacrylsäure sind diejenigen mit 1 bis 18 Kohlenstoffatomen im Alkylrest bevorzugt, insbesondere Methyl-, Ethyl-, n-Butyl-, Isobutyl-, Pentyl-, 2-Ethylbutyl-, n-Hexyl-, Heptyl-, n-Octyl-, Isooctyl-, 2-Ethylhexyl-, n-Decyl-, Isodecyl-, n-Dodecyl- und Stearylacrylat bzw. -methacrylat. Daneben können weitere Comonomere am Aufbau des Polymers/Copolymers beteiligt sein. Beispiele sind Acryl- und/oder Methacrylamid, Hydroxyalkylester und Polyalkylenglykolester der Acryl- und/oder Methacrylsäure, stickstoffhaltige Monomere der Acryl- und/oder Methacrylsäure oder deren Salze, Ethylen, Vinylacetat, Vinylpropionat, Vinylbutyrat, Vinylpyrrolidon, Vinylchlorid, Vinyltoluol, Acrylnitril, Styrol und dergleichen.

Zur Herstellung eines erfindungsgemäßen Systems wird vorteilhaft ein Acrylat-Copolymer eingesetzt, welches 2-Ethylhexylacrylat, Vinylacetat, Hydroxyethylacrylat und Glycidylmethacrylat enthält.

Um die therapeutische Wirksamkeit sicherzustellen wird Glycerol als Enhancer eingesetzt. Hierzu muß Glycerol in einer größeren Menge in die Matrix eingearbeitet werden. Problematisch ist, daß Glycerol in größeren Mengen die Kohäsion der Matrix mindert. Um diesem Effekt entgegenzuwirken, wird der Acrylatgrundlage ein weiteres Polymer zugefügt, das für sich allein nichtklebend ist, aber über sehr gute filmbildende Eigenschaften verfügt. Dieses filmbildende Polymer hat einen positiven Einfluß auf die Kohäsion der Matrix. Als gut geeignet erweisen sich Polymere auf der Basis von Polyacryl- bzw. Polymethacrylsäure und deren Estern.

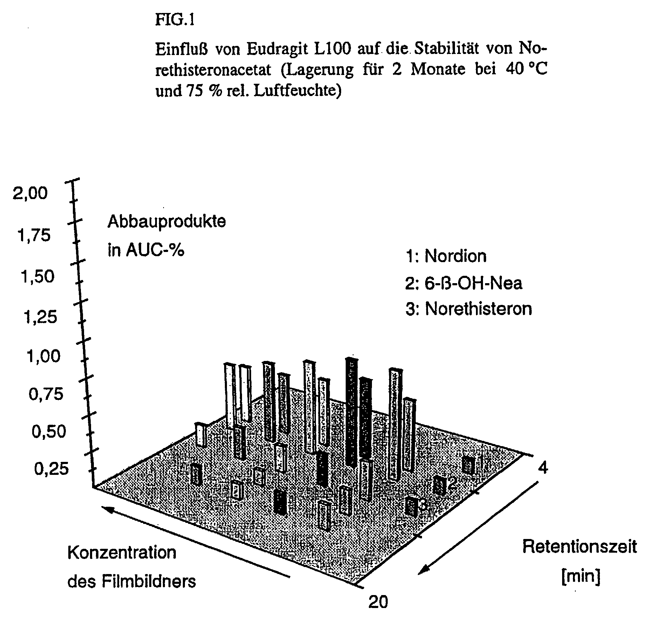

Zur Herstellung eines erfindungsgemäßen Systems wird ein filmbildendes Acrylat-Copolymer mit 10-90 Gew.-% Methacrylsäure und 10-90 Gew.-% Methylmethacrylat eingesetzt. Überraschend ist, daß der Zusatz der sauer reagierenden Komponente nicht zu einem verstärkten Abbau des eingearbeiteten Hormons mit 3-Keto-4-en Partialstruktur führt. Dies steht im klaren Widerspruch zu den in der WO 97/03629 beschriebenen Versuchsergebnissen. Der Einsatz des genannten Polymers trägt im Gegenteil eher noch zur Stabilität bei. In FIG.1 ist der Einfluß von Eudragit L100 auf die Bildung von Abbauprodukten des Norethisteronacetats dargestellt. Die mit den Nummern 1-3 gekennzeichneten Balken stellen bekannte Abbauprodukte dar (Nordion, 6-beta-Hydroxynorethisteronacetat und Norethisteron). Die z-Achse gibt die dazu gehörigen Retentionszeiten an, korrespondierend zur durchgeführten RP-HPLC. Die y-Achse gibt die prozentualen Werte für die gebildeten Abbauprodukte in Bezug auf die Gesamtfläche im RP-HPLC Chromatogramm an. Die zweite Reihe von rechts auf der x-Achse zeigt, daß in einer Formulierung, die kein Eudragit L100 enthält, die meisten Abbauprodukte gebildet werden. Mit zunehmenden Gehalt an Eudragit L100 (von rechts nach links, von 1-10 % Eudragit L100, bezogen auf die Trockenmasse) nimmt die Summe der Abbauprodukte ab. Die TTS wurden für 2 Monate bei 40 °C und 75 % relativer Luftfeuchte gelagert.

Zur Verbesserung der Kohäsion werden vorteilhaft Zusatzstoffe, vorzugsweise Metallionen wie Aluminium oder Titan eingearbeitet.

Wenn die Klebkraft der Matrix durch den Zusatz des filmbildenden Polymers reduziert ist, kann durch Beimischung von stark klebrigmachenden Harzen wirksam Abhilfe geschaffen werden. Als klebrigmachende Mittel können Kolophoniumharze, Polyterpenharze, Petroleumharze, Cumaron-Inden-Harze, Terpenphenolharze, Kohlenwasserstoffharze, flüssige Polybutenharze und dergleichen bei der Herstellung der erfindungsgemäßen Zusammensetzung verwendet werden.

Nachfolgend wird die Erfindung anhand eines Beispiels näher erläutert:

### BEISPIEL:

1,4 g Estradiol, 7,5 g Norethisteronacetat, 105 g Acrylatklebstoff (Durotak® 387-2287), 46 g Klebstoffharz (z.B. Hercolyn® DE), 10 g Eudragit L100, 30 g Glyzerin, 15 g Acetylaceton und 0,1 g Aluminiumacetylacetonat (4 Gew.% in Ethylacetat) wurden gemischt. Die Lösung wurde mit einer Naßschichtdicke von 200 µm mit Hilfe einer Rakel auf eine silikonisierte Polyesterfolie (z.B. Hostaphan®) aufgetragen. Der feuchte Film wurde für 30 Minuten bei 50 °C getrocknet und anschließend mit einer Polyesterfolie (z.B. Hostaphan®) laminiert. Das Flächengewicht eines derartig hergestellten Klebefilms betrug ca. 80,4 g/m². Aus dem Laminat wurden mittels geeigneter Stanze TTS der gewünschten Größe ausgestanzt und zur Untersuchung der Stabilität bei verschiedenen Temperaturen eingelagert.

## Patentansprüche

1. Medizinische selbstklebende Zusammensetzung zur transdermalen Applikation eines Estrogens in Kombination mit einem Gestagen, enthaltend
(a) 25-90 Gew.-% eines selbstklebenden Acrylat-Copolymers,
(b) 1-15 Gew.-% eines filmbildenden Acrylat-Copolymers, das 10-90 Gew.-% Methacrylsäure und 10-90 Gew.-% Methylmethacrylat enthält,
(c) 1-30 Gew.-% die Klebrigkeit erhöhende Klebharzzusätze,
(d) 0,2-2,0 Gew.-% Estrogen und
(e) 1-5 Gew.-% Gestagen.

2. Zusammensetzung nach Anspruch 1, **dadurch gekennzeichnet, daß** das selbstklebende Acrylat-Copolymer 2-Ethylhexylacrylat, Vinylacetat, Hydroxyethylacrylat und Glycidylmethacrylat enthält.

3. Zusammensetzung nach Anspruch 1, **dadurch gekennzeichnet, daß** der die Klebrigkeit erhöhender Zusatz ein Derivat des Kolophoniums ist.

4. Zusammensetzung nach Anspruch 1, **dadurch gekennzeichnet, daß** der die Klebrigkeit erhöhender Zusatz ein Polyterpenharz ist.

5. Zusammensetzung nach Anspruch 1, **dadurch gekennzeichnet, daß** ein die Kohäsion steigernder Zusatz enthalten ist.

6. Zusammensetzung nach Anspruch 5, **dadurch gekennzeichnet, daß** es sich bei dem kohäsionssteigernden Zusatz um ein Metallion, vorzugsweise des Aluminiums oder Titans, handelt.

7. Zusammensetzung nach Anspruch 1, **dadurch gekennzeichnet, daß** das Estrogen ein Derivat des Estradiols, insbesondere 17-beta-Estradiol ist.

8. Zusammensetzung nach Anspruch 1, **dadurch gekennzeichnet, daß** das Gestagen ein Derivat des Norethisterons, insbesondere Norethisteronacetat, ist.

9. Zusammensetzung nach Anspruch 1, **dadurch gekennzeichnet, daß** sie 1-20 Gew.-% wasserfreies Glycerin enthält.

10. Verwendung der Zusammensetzung nach einem der Ansprüche 1 bis 7 zur Herstellung eines Arzneimittels zur Prophylaxe und Behandlung der klimakterischen Beschwerden.

## Claims

1. Medicinal self-adhesive composition for transdermal application of an estrogen in combination with a gestagen, containing
(a) 25-90%-wt. of a self-adhesive acrylate copolymer,
(b) 1-15%-wt. of a film-forming acrylate copolymer which contains 10-90%-wt. of methacrylic acid and 10-90%-wt. of methyl methacrylate,
(c) 1-30%-wt. of tack-increasing adhesive resin additives,
(d) 0.2-2.0%-wt. of estrogen and
(e) 1-5%-wt. of gestagen.

2. Composition according to Claim 1, **characterized in that** the self-adhesive acrylate copolymer contains 2-ethylhexyl acrylate, vinyl acetate, hydroxyethyl acrylate and glycidyl methacrylate.

3. Composition according to Claim 1, **characterized in that** the tack-increasing additive is a derivative of colophony.

4. Composition according to Claim 1, **characterized in that** the tack-increasing additive is a polyterpene resin.

5. Composition according to Claim 1, **characterized in that** a cohesion-increasing additive is contained.

6. Composition according to Claim 5, **characterized in that** the cohesion-increasing additive is a metal ion, preferably of aluminium or titanium.

7. Composition according to Claim 1, **characterized in that** the estrogen is a derivative of estradiol, especially 17-beta-estradiol.

8. Composition according to Claim 1, **characterized in that** the gestagen is a derivative of norethisterone, especially norethisterone acetate.

9. Composition according to Claim 1, **characterized in that** it contains 1-20%-wt. of anhydrous glycerin.

10. The use of the composition according to any one of Claims 1 to 7 for producing a pharmaceutical product for prophylaxis and treatment of climacteric complaints.

## Revendications

1. Composition médicale auto-adhésive pour l'application transdermique d'un oestrogène en combinaison avec un progestatif, contenant
(a) un copolymère d'acrylate auto-adhésif à concurrence de 25 à 90 % en poids ;
(b) un copolymère d'acrylate filmogène à concurrence de 1 à 15 % en poids, qui contient de l'acide méthacrylique à concurrence de 10 à 90 % en poids et du méthacrylate de méthyle à concurrence de 10 à 90 % en poids ;
(c) des additifs de résine adhésive augmentant l'adhésivité à concurrence de 1 à 30 % en poids ;
(d) un oestrogène à concurrence de 0,2 à 2,0 % en poids ; et
(e) un progestatif à concurrence de 1 à 5 % en poids.

2. Composition selon la revendication 1, **caractérisée en ce que** le copolymère d'acrylate auto-adhésif contient de l'acrylate de 2-éthylhexyle, de l'acétate de vinyle, de l'acrylate d'hydroxyéthyle et du méthacrylate de glycidyle.

3. Composition selon la revendication 1, **caractérisée en ce que** l'additif augmentant l'adhésivité est un dérivé du colophane.

4. Composition selon la revendication 1, **caractérisée en ce que** l'additif augmentant l'adhésivité est une résine polyterpénique.

5. Composition selon la revendication 1, **caractérisée en ce qu'**elle contient un additif augmentant la cohésion.

6. Composition selon revendication 5, **caractérisée en ce qu'**il s'agit, en ce qui concerne l'additif augmentant la cohésion, d'un ion métallique, de préférence de l'aluminium ou du titane.

7. Composition selon la revendication 1, **caractérisée en ce que** l'oestrogène est un dérivé de l'oestradiol, en particulier le 17-bêta-oestradiol.

8. Composition selon la revendication 1, **caractérisée en ce que** le progestatif est un dérivé de la noréthistérone, en particulier l'acétate de noréthistérone.

9. Composition selon la revendication 1, **caractérisée en ce qu'**elle contient du glycérol anhydre à concurrence de 1 à 20 % en poids.

10. Utilisation de la composition selon l'une quelconque des revendications 1 à 7, pour la préparation d'un médicament destiné à la prophylaxie et au traitement des doléances climactériques.
